# FASCICULE DE BREVET EUROPEEN

(11) **EP 1 345 540 B1**
(45) Date de publication et mention de la délivrance du brevet: **12.05.2004**
(21) Numéro de dépôt: 01271914.2
(22) Date de dépôt: 12.12.2001
(51) Int. Cl.: A61B 17/122

(54) **DISPOSITIF DE CLAMPAGE POUR STRUCTURE ANATOMIQUE**
KLEMMVORRICHTUNG FÜR EINE ANATOMISCHE STRUKTUR
CLAMPING DEVICE FOR ANATOMICAL STRUCTURE

(30) Priorité: 26.12.2000 EP 00204750
(43) Date de publication de la demande: 24.09.2003
(73) Titulaire: Cardio Life Research S.A., 1348 Louvain la Neuve (BE)
(72) Inventeur: DE CANNIERE, Bernard, B-1050 Bruxelles (BE)
(74) Mandataire: Van Straaten, Joop
(86) Numéro de dépôt international: PCT/BE2001/000211
(87) Numéro de publication internationale: WO 2002/051321

(56) Documents cités:
- US-A- 5 618 307

## Description

L'invention concerne les dispositifs de clampage pour structures anatomiques caves tubulaires, telles que, essentiellement, les vaisseaux sanguins, mais aussi trachées, intestins, etc.

### Arrière-plan technologique de l'invention

La chirurgie cardiaque requiert le plus souvent un arrêt cardiaque afin d'obtenir un site opératoire immobile et exsangue permettant un geste chirurgical précis et délicat. Ceci nécessite : 1) le recours à la circulation extra-corporelle (CEC) afin de perfuser les organes systémiques (cerveau, foie, reins etc.) avec du sang oxygéné pendant la période ou le coeur est arrêté.

2) Le clampage de l'aorte qui consiste à obturer le vaisseau par une pince externe qui est interposée entre la canule artérielle permettant la circulation extra-corporelle et l'orifice des artères coronaires. Cette manoeuvre isole la circulation coronaire du flux sanguin apporté par la CEC et donc permet d'arrêter le coeur.

3) La cardioplégie : injection d'une solution dans le réseau des artères coronaires pour protéger le coeur lui-même pendant la période d'arrêt.

La mise en place de la circulation extra-corporelle (CEC), le clampage et la cardioplégie nécessitent classiquement la découpe et l'écartement du sternum (sternotomie). La sternotomie est une voie d'abord chirurgicale délabrante et porteuse de complications post opératoires fréquentes pour les patients.

Par ailleurs, le clampage de l'aorte est une opération considérée comme délicate et risquée du fait, notamment, de la proximité de l'artère pulmonaire, dont la texture est connue comme extrêmement fragile.

En outre, le pincement de l'artère à l'aide d'un clamp classique est source d'embolisation de matériel athéromateux qui tapisse dans la plupart des cas la paroi interne du vaisseau.

Depuis plusieurs années, la chirurgie cardiaque développe des techniques alternatives visant à procurer une moindre agression pour les patients. L'éviction de la sternotomie est une de ces approches. Dans ce cas, l'intervention est conduite par des mini-incisions permettant l'introduction d'instruments endoscopiques.

Dans ce type d'intervention, quand le coeur doit être arrêté, un ballon gonflable est introduit à l'intérieur de l'artère aorte, à la sortie du coeur, sous contrôle échographique ou fluoroscopique, pour boucher l'aorte de l'intérieur du vaisseau.

Ce système présente de nombreux inconvénients dont, notamment, son coût, qui en limitent l'utilisation à un nombre de cas marginaux. En outre, on déloge également, par cette technique, des particules entraînées par le flux sanguin.

Le document US-A-5 618 307 décrit un clamp chirurgical qui comprend deux mâchoires allongées et un mandrin enfilable sur les mâchoires apte à rapprocher l'un de l'autre les axes des deux mâchoires

On a donc recherché une solution alternative permettant à un grand nombre de patients de subir en toute sécurité une intervention cardiaque aussi peu invasive que possible.
L'objet de l'invention est un clamp chirurgical qui comprend
- un guide flexible;
- deux mâchoires formées chacune d'un membre allongé percé d'un canal longitudinal et présentant une extrémité distale et une extrémité proximale, les dites mâchoires étant enfilables sur le guide avec leur extrémité proximale tournée chacune vers une des extrémités du guide;
- un mandrin enfilable sur les deux extrémités du guide et sur les mâchoires et apte à rapprocher l'un de l'autre les axes des deux mâchoires.

La fermeture des mâchoires est assurée de préférence par une tige de commande enfilée sur les deux extrémités du guide en amont du mandrin.

Avantageusement, une gaine intermédiaire est mise en place à l'aide du guide. Cette gaine souple peut être positionnée dans le thorax en regard d'une structure vasculaire encerclée par le guide.

Les mors présentent de préférence une section malléable de côté de leur extrémité proximale.

Suivant un mode de réalisation avantageux, le mandrin comprend un seul canal longitudinal, l'extrémité distale de cette cavité étant profilée de façon à entraîner le rapprochement des axes des deux mâchoires par un déplacement relatif.

Suivant un autre mode de réalisation avantageux le mandrin comprend deux canaux longitudinaux, chacune des deux mâchoires étant enfilées dans un des deux canaux.

Les mâchoires sont pourvues de préférence, près de leur extrémité distale, de mors souples.

Suivant un mode de réalisation avantageux, les mâchoires sont serrées par un dispositif de solidarisation à articulation unique ou multiples.
La fermeture des mâchoires est assurée de préférence par une commande enfilée sur les deux extrémités du guide en amont du mandrin.

la partie proximale des mâchoires est munie avantageusement de cannelures coopérant avec un mécanisme supporté par le mandrin.

Le mécanisme supporté par le mandrin comprend de façon préférée au moins un linguet muni d'un bec et d'une partie résiliente amenant le dit bec en contact avec les cannelures des mâchoires

On peut utiliser l'objet de l'invention dans une méthode de clampage d'un vaisseau sanguin comprenant les opérations suivantes
- ménager un trou de port dans un organisme à sang chaud
- introduire un guide souple dans le dit trou de port
- faire ressortir l'extrémité distale de ce guide par le trou de port
- enfiler sur chacune des extrémités du guide souple un membre allongé muni d'un canal longitudinal, d'une partie distale et d'une partie proximale
- amener les extrémités distales de ces deux membres allongés de part et d'autre d'un vaisseau sanguin à clamper
- enfiler sur les extrémités proximales de ces membres allongés un mandrin creux
- faire remonter le mandrin creux vers l'extrémité distale des membres allongés, entraînant ainsi le rapprochement des membres allongés l'un vers l'autre et le pincement du vaisseau sanguin.

Dans cette méthode de clampage le vaisseau sanguin peut être l'artère aorte, la dite méthode comprenant en outre les opérations suivantes :
- ménager un trou de port dans un thorax
- pratiquer une incision dans le péricarde
- introduire le guide souple dans le sinus transverse de Theile

L'avantage du clamp de l'invention est que l'on obtient un clampage efficace et puissant par une incision minime, de l'ordre du centimètre, rendant par là l'intervention extrêmement peu invasive.

Le dispositif de l'invention s'applique de façon particulièrement avantageuse au clampage de l'aorte grâce à l'utilisation de l'espace anatomique du sinus transverse de Theile comme guide naturel.

Un autre avantage est que le risque d'abîmer un organe adjacent est réduit à un minimum. En particulier dans le cas d'interventions cardiaques, le risque de déchirer l'artère pulmonaire devient pratiquement négligeable, puisqu'il n'est plus nécessaire de pratiquer la dissection du plan virtuel entre les deux vaisseaux.

Le clamp de l'invention peut être utilisé aussi bien pour des structures vasculaires intra et extra thoraciques qu'à d'autres structures anatomiques dont notamment les intestins. Il peut également être utilisé comme davier pour manipuler des os.
D'autres particularités et avantages de l'invention ressortiront de la description ci-après de modes de réalisation particuliers de l'invention, référence étant faite aux dessins annexés dans lesquels :
La Fig. 1 est une vue schématique en perspective du clampage d'un coeur humain dans le cas d'une opération avec sternotomie.
La Fig. 2 est une vue avec arrachement d'une opération du coeur avec mini-incision intercostale.
Les Fig. 3 à 7 sont des vues schématiques du principe de clampage du dispositif de l'invention.
La Fig. 8 est une vue en coupe d'une paire de mors du clamp de l'invention.
La Fig. 9 est une vue éclatée d'une forme de réalisation du clamp de l'invention.

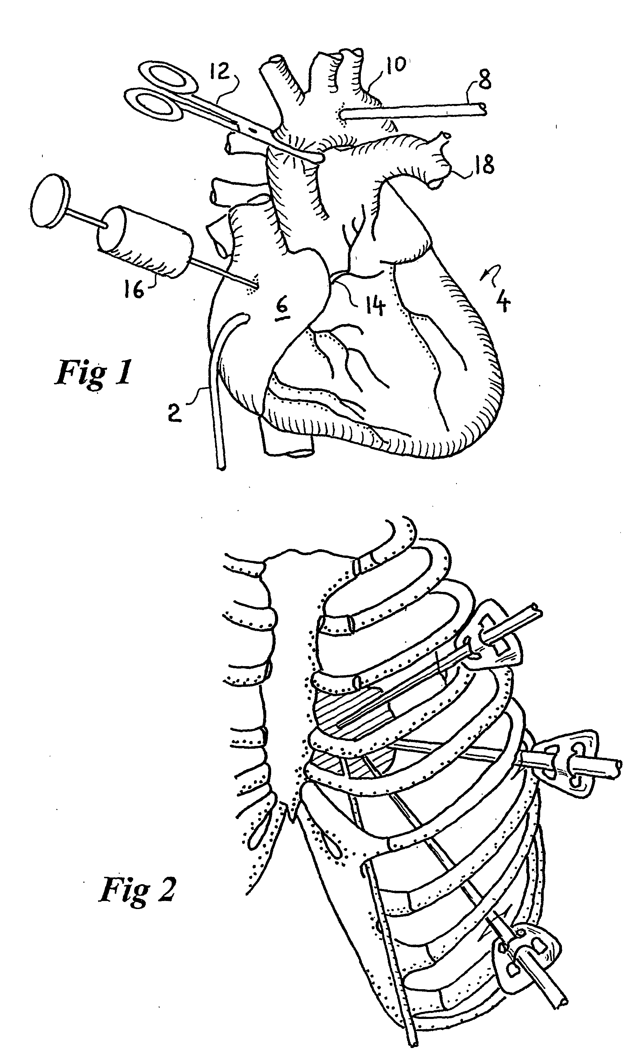

La Fig. 1 montre les différentes opérations préparatoires à une intervention cardiaque classique, afin d'obtenir un champ opératoire exsangue et immobile.

Le sang veineux (pauvre en oxygène) est dérivé par une canule 2 lors de son entrée dans le coeur 4 par l'oreillette droite 6 vers une machine coeur-poumons (non représentée) qui le réoxygène et le débarrasse de son CO₂. Le sang artificiellement oxygéné est ensuite réinjecté par une deuxième canule 8 à hauteur de l'aorte 10 dans le circuit artériel du patient, court-circuitant ainsi le coeur 4 et la circulation pulmonaire afin de permettre le geste opératoire intra ou extra-cardiaque.

Le coeur 4 peut alors être arrêté pour obtenir un champ opératoire exsangue et immobile.

L'arrêt cardiaque est classiquement obtenu par deux manoeuvres concomitantes :
- Le clampage aortique;
- L'injection d'une solution de cardioplégie dans la circulation coronaire.

Le clampage de l'aorte 10 consiste à obturer le vaisseau par une pince 12 externe qui est interposée entre la canule artérielle 8 de la circulation extra-corporelle et l'orifice des artères coronaires 14. Cette manoeuvre isole la circulation coronaire du flux sanguin engendré par la CEC.

Une solution de cardioplégie peut dès lors être injectée par un organe d'injection 16 dans la circulation coronaire pour "paralyser" le coeur 4 dans le but de permettre au chirurgien un geste plus précis qu'il ne le serait sur des structures anatomiques en mouvement.

La Fig. 2 montre une autre approche connue, dans laquelle une intervention cardiaque est menée via une ou plusieurs incisions de l'ordre du centimètre permettant l'introduction d'instruments endoscopiques.

Le problème qui se pose dans ce cas est d'interrompre de façon sûre la circulation du sang dans l'aorte en évitant les inconvénients inhérents à l'introduction d'un ballonnet.

Comme il a été dit plus haut, il est impossible d'avoir recours à une pince classique dont la dimension des mâchoires et leur course sont hors de proportion avec celles des incisions intercostales pratiquées.

L'avantage majeur du système selon l'invention est de permettre le clampage sans ouverture du thorax au prix, qui plus est, d'un risque moindre de traumatisme de l'artère pulmonaire et d'embolie.

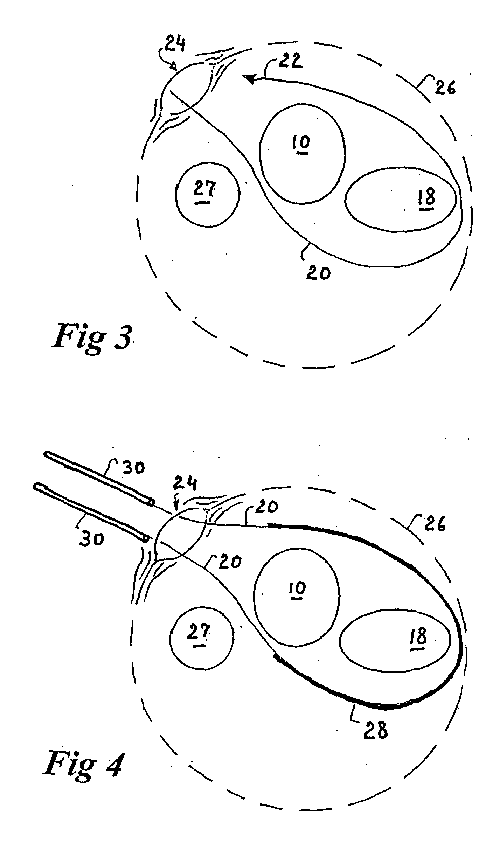

Le dispositif de clampage de l'invention et ses différents composants seront décrits en se référant à la succession des Fig. 3 à 7.

Un guide 20 souple, éventuellement muni d'une extrémité 22 dirigeable, est introduit dans le thorax par voie gauche ou droite dans un trou de port, puis par une incision 24 dans le péricarde dans le sinus transverse de Theile, espace anatomique virtuel naturel 26 recouvert de séreuse péricardique. Il suffit de pousser le guide souple 20 pour qu'il réapparaisse en encerclant les vaisseaux à clamper. En l'occurrence, il s'agit des gros vaisseaux de la base du coeur, c'est-à-dire l'aorte 10 et l'artère pulmonaire 18. Le guide permet d'éviter d'autres organes (tels qu'ici, la veine cave supérieure 27). L'extrémité 22 de ce guide 20 est alors recapturée par une contre incision si nécessaire et ressorti par le même trou de port (Figure 3).

Suivant les caractéristiques mécaniques du guide et la nature des vaisseaux à enserrer, une gaine intermédiaire 28 (Figure 4) est enfilée sur le guide souple 20. Suivant une autre technique, cette gaine intermédiaire 28 est accrochée à une des extrémités du guide et hâlée en place.

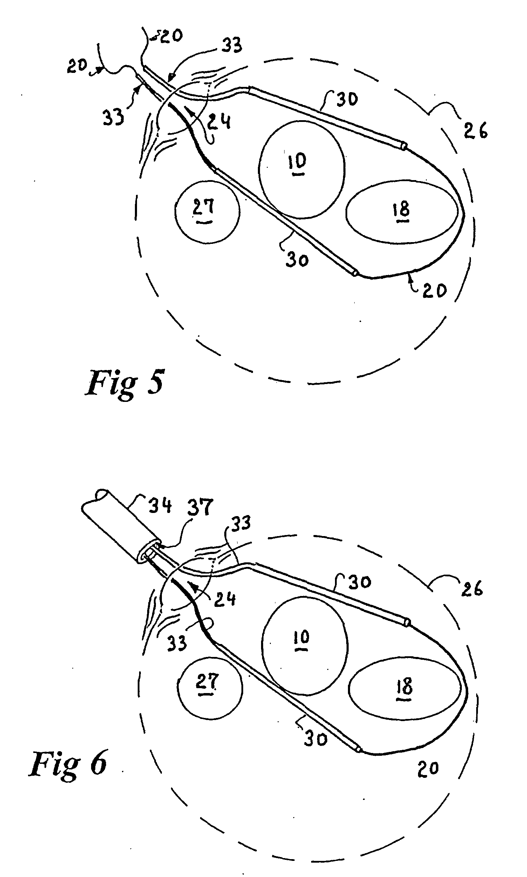

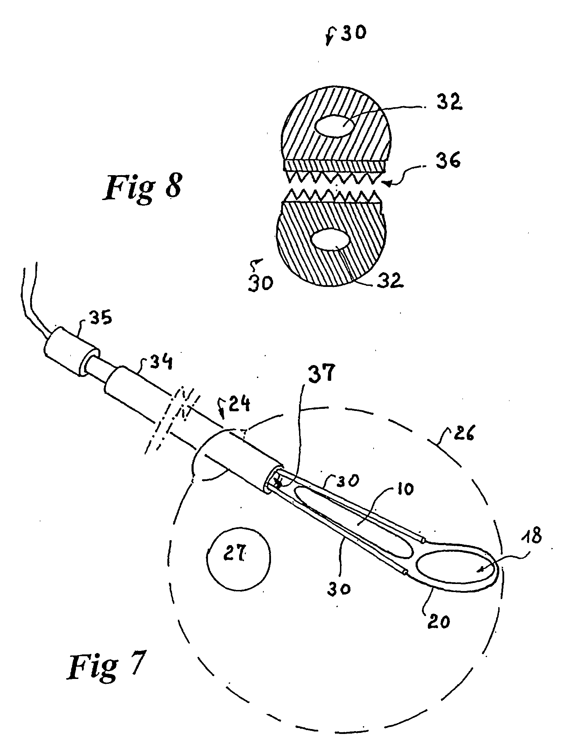

L'opérateur enfile ensuite (Fig. 4) sur chacune des extrémités du guide 20 (ou de la gaine intermédiaire 28)une mâchoire 30 formée d'un membre allongé percé d'un canal longitudinal 32 (visible à la Fig. 8), ces mâchoires 30 sont déplacées de façon à ce que leurs extrémités distales viennent se placer de part et d'autre au vaisseau à enserrer (en l'occurrence, l'aorte 10). Les mâchoires 30 représentées aux Fig. 5 et 6 diffèrent des mâchoires 30 de la Fig. 4 en ce qu'elles comprennent chacune, à leur extrémité proximale, une partie malléable 33.

Les mâchoires 30 étant en place, l'opérateur enfile sur les deux extrémités du guide souple 20 un mandrin 34 creux (Fig. 6) et fait remonter ce mandrin 34 vers l'extrémité distale des mâchoires 30 (Fig. 7). Les mâchoires 30 s'alignent alors chacune suivant l'axe du mandrin 34 et se rapprochent l'une de l'autre, entraînant un pincement délicat et progressif de l'aorte 10.

Le dispositif entraînant le pinçage peut être agencé de différentes façons :

Dans sa conception la plus simple, la partie proximale des mâchoires comprend une surface conique qui coopère avec l'embouchure du canal longitudinal 37 du mandrin 34, entraînant un alignement des mâchoires. Ce canal 37 peut être simple ou double. Les mâchoires peuvent également être munies de cannelures coopérant avec un pas de vis ménagé à la surface interne du mandrin 34.

Suivant un autre agencement (Figure 7), une pièce 35 mobile par rapport au mandrin 34 est montée sur les deux extrémités proximales des mâchoires 34. Par déplacement relatif de cette pièce 35 avec le mandrin 34, notamment par l'intermédiaire d'une partie filetée, on obtient un serrage ou un desserrage des mâchoires 30.

La Fig. 8 montre, en coupe, la partie distale des mâchoires 30. Dans ce mode,de réalisation, celles-ci sont pourvues de mors souples 36 de façon à répartir la pression sur l'organe saisi. On distingue sur cette coupe le canal longitudinal 32 permettant de faire passer le guide 20.

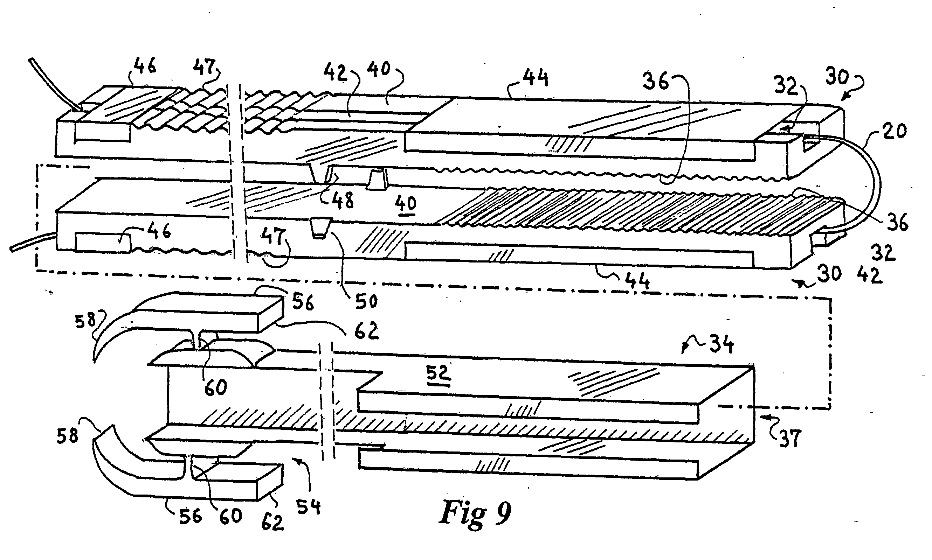

La Fig. 9 montre, démontée, une forme de réalisation du clamp de l'invention. Chaque membre formant une mâchoire 30 comprend une languette 40 en matériau plastique. Les faces de ces languettes tournées l'une vers l'autre, légèrement dentelées, forment les mors 36. Le dos de chaque languette 40 comporte une rainure longitudinale 42 destinée à recevoir le guide souple 20.

La partie distale des languettes 40 est enserrée dans un profilé métallique en U, 44 qui ferme le canal longitudinal 32 et confère aux mâchoires 30 la rigidité nécessaire pour exercer le pinçage. Une deuxième pièce en U, 46 ferme un autre segment du canal longitudinal près de l'autre extrémité de chaque languette 40. Le dos de la partie proximale des languettes est crénelé 47.

Chacune des languettes 40 porte à sa partie médiane en ergot 48 et une alvéole 50 destinés à s'engrener dans les parties correspondantes 50, 48 de l'autre languette 40, de façon à empêcher tout déplacement relatif des mâchoires dès que leurs extrémités sont insérées dans le mandrin 34.

Dans cette forme de réalisation, le mandrin 34 est formé lui-même, pour l'essentiel, d'une pièce métallique 52 repliée sur elle-même. Du côté distal, cette pièce métallique présente une section en C qui permet d'y introduire et de guider les mâchoires 30.

La partie proximale du mandrin 34 supporte un mécanisme permettant d'ajuster la position relative des mâchoires 30 et du mandrin 34. Ce mécanisme comprend un double linguet 56, 56. Au repos, le bec 58 de chacun de ces linguets 56 est engagé dans le crénelage 47 proximal des languettes 40 par une partie faisant ressort 50. Une pression sur le talon 62 des linguets 56 libère les languettes 40, ce qui permet aux mâchoires 30 de coulisser librement par rapport au mandrin 34.

## Revendications

1. Clamp chirurgical qui comprend
- un guide (20) flexible;
- deux mâchoires (30) formées chacune d'un membre allongé percé d'un canal longitudinal (32) et présentant une extrémité distale et une extrémité proximale, les dites mâchoires (30) étant enfilables sur le guide (20) avec leur extrémité proximale tournée chacune vers une des extrémités du guide;
- un mandrin (34) enfilable sur les deux extrémités du guide (20) et apte à rapprocher l'un de l'autre les axes des deux mâchoires (30).

2. Clamp chirurgical suivant la revendication 1 **caractérisé en ce qu'**une gaine intermédiaire (28) est mise en place à l'aide du guide (20).

3. Clamp chirurgical suivant l'une quelconque des revendications précédentes **caractérisé en ce que** les mâchoires (30) présentent une section malléable (33) du côté de leur extrémité proximale.

4. Clamp chirurgical suivant l'une quelconque des revendications précédentes **caractérisé en ce que** le mandrin (34) comprend un seul canal longitudinal (37), l'extrémité distale de ce canal (32) étant profilée de façon à entraîner le rapprochement des axes des deux mâchoires (30) par un déplacement relatif.

5. Clamp chirurgical suivant l'une quelconque des revendications 1 à 3 **caractérisé en ce que** le mandrin (34) comprend deux canaux longitudinaux (37), chacune des deux mâchoires (30) étant enfilées dans un des deux canaux (37).

6. Clamp chirurgical suivant l'une quelconque des revendications précédentes **caractérisé en ce que** les mâchoires (30) sont pourvues près de leur extrémité distale de mors souples (36).

7. Clamp chirurgical suivant l'une quelconque des revendications précédentes **caractérisé en ce que** les mâchoires (30) sont serrées par un dispositif de solidarisation à articulation unique ou multiples.

8. Clamp chirurgical suivant l'une quelconque des revendications 1 à 6 **caractérisé en ce que** la fermeture des mâchoires (30) est assurée par une commande (35) enfilée sur les deux extrémités du guide (20) en amont du mandrin (34).

9. Clamp chirurgical suivant l'une quelconque des revendications précédentes **caractérisé en ce que** la partie proximale des mâchoires (30) est munie de cannelures (47) coopérant avec un mécanisme supporté par le mandrin (34).

10. Clamp chirurgical suivant la revendication 9 **caractérisé en ce que** le mécanisme supporté par le mandrin (34) comprend au moins un linguet (56) muni d'un bec (58) et d'une partie résiliente (60) amenant le dit bec (58) en contact avec les cannelures (47) des mâchoires (30).

## Claims

1. A surgical clamp which comprises
- a flexible guide (20);
- two jaws (30) each formed of an elongate member pierced with a longitudinal canal (32) and having a distal end and a proximal end, it being possible for said jaws (30) to be slipped over the guide with each of their proximal end facing toward one of the ends of the guide;
- a mandrel (34) that can be slipped over the two ends of the guide (20) and which is able to bring the axes of the two jaws (30) closer together.

2. Surgical clamp as claimed in claim 1, **characterized in that** an intermediate sheath (28) is fitted using the guide (20).

3. Surgical clamp as claimed in either of the preceding claims, **characterized in that** the jaws (30) have a malleable section (33) towards their proximal end.

4. Surgical clamp as claimed in any one of the preceding claims, **characterized in that** the mandrel (34) comprises a single longitudinal canal (37), the distal end (32) of this canal being shaped in such a way as to cause the axes of the two jaws (30) to be moved closer together by relative displacement.

5. Surgical clamp as claimed in any one of claims 1 to 3, **characterized in that** the mandrel (34) comprises two longitudinal canals (37), each of the two jaws (30) being slipped into one of the two canals (37).

6. Surgical clamp as claimed in any one of the preceding claims, **characterized in that** the jaws (30) are provided with flexible jaw elements (36) near their distal end.

7. Surgical clamp as claimed in any one of the preceding claims, **characterized in that** the jaws (30) are clamped using a securing device with a single articulation or multiple articulations.

8. Surgical clamp as claimed in any one of claims 1 to 6, **characterized in that** the jaws (30) are closed by a control (35) slipped over the two ends of the guide (20) upstream of the mandrel (34).

9. Surgical clamp as claimed in any one of the preceding claims, **characterized in that** the proximal part of the jaws (30) is equipped with ribbing (37) collaborating with a mechanism supported by the mandrel (34).

10. Surgical clamp as claimed in claim 9, **characterized in that** the mechanism supported by the mandrel (34) comprises at least one pawl (56) equipped with a nose (58) and with a resilient part (60) bringing said nose (58) into contact with the ribbing (47) of the jaws (30).

## Patentansprüche

1. Chirurgische Klammer, die folgendes umfasst:
- eine flexible Führung (20),
- zwei Backen (30), die jeweils von einem länglichen Element gebildet werden, das von einem länglichen Kanal (32) durchbohrt wird und ein distales Ende und ein proximales Ende aufweist, wobei die Backen (30) mit ihrem proximalen, jeweils zu einem der Enden der Führung gewendeten Ende auf die Führung (20) auffädelbar sind,
- ein auf die zwei Enden der Führung (20) aufschiebbares Futter (34), das in der Lage ist, die Achsen der zwei Backen (30) einander anzunähern.

2. Chirurgische Klammer nach Anspruch 1, **dadurch gekennzeichnet, dass** mittels der Führung (20) eine Zwischenscheide (28) platziert wird.

3. Chirurgische Klammer nach einem der vorhergehenden Ansprüche, **dadurch gekennzeichnet, dass** die Backen (30) einen verformbaren Querschnitt (33) auf der Seite ihres proximalen Endes aufweisen.

4. Chirurgische Klammer nach einem der vorhergehenden Ansprüche, **dadurch gekennzeichnet, dass** das Futter (34) einen einzelnen Längskanal (37) umfasst, wobei das distale Ende dieses Kanals (32) so profiliert ist, dass die Annäherung der Achsen der zwei Backen (30) durch eine Relativverschiebung herbeigeführt wird.

5. Chirurgische Klammer nach einem der Ansprüche 1 bis 3, **dadurch gekennzeichnet, dass** das Futter (34) zwei Längskanäle (37) umfasst, wobei jede der Backen (30) in einen der zwei Kanäle (37) eingefädelt wird.

6. Chirurgische Klammer nach einem der vorhergehenden Ansprüche, **dadurch gekennzeichnet, dass** die Backen (30) nahe ihrem distalen Ende mit flexiblen Spannbacken (36) versehen sind.

7. Chirurgische Klammer nach einem der vorhergehenden Ansprüche, **dadurch gekennzeichnet, dass** die Backen (30) durch eine Befestigungsvorrichtung mit einem einzelnen oder meheren Gelenken zusammengedrückt werden.

8. Chirurgische Klammer nach einem der Ansprüche 1 bis 6, **dadurch gekennzeichnet, dass** für das Schließen der Backen (30) durch eine Steuerung (35) gesorgt wird, die auf die zwei Enden der Führung (20) stromaufwärts von dem Futter (34) aufgefädelt ist.

9. Chirurgische Klammer nach einem der vorhergehenden Ansprüche, **dadurch gekennzeichnet, dass** der proximale Teil der Backen (30) mit Riefelungen (47) versehen ist, die mit einem durch das Futter (34) getragenen Mechanismus zusammenwirken.

10. Chirurgische Klammer nach Anspruch 9, **dadurch gekennzeichnet, dass** der von dem Futter (34) getragene Mechanismus zumindest eine Sperrklinke (56) umfasst, die mit einer Spitze (58) und einem elastischen Teil (60) versehen ist, das die Spitze (58) in Kontakt mit den Riefelungen (47) der Backen (30) bringt.
